(19) Europäisches Patentamt
European Patent Office
Office européen des brevets

(11) EP 1 033 358 B1

(12) EUROPEAN PATENT SPECIFICATION

(45) Date of publication and mention of the grant of the patent:
**08.06.2005 Bulletin 2005/23**

(51) Int Cl.[7]: **C07C 2/66**

(21) Application number: **99301516.3**

(22) Date of filing: **01.03.1999**

(54) **Control of diarylalkane by-product formation in alkylation of aromatics by olefins using zeolite beta catalyst**

Beherrschung der Bildung von Diarylalkanen als Nebenprodukt in einem Verfahren zur Alkylierung von aromatischen Kohlenwasserstoffen mit Olefinen mit Verwendung von Zeolit Beta als Katalysator

Orientation de la formation de diarylakanes comme produit secondaire dans un procédé d'alkylation d'hydrocarbures aromatiques avec des oléfines avec utilisation de zéolite beta comme catalyseur

(84) Designated Contracting States:
**AT BE DE ES FI FR GB GR IT NL PT SE**

(43) Date of publication of application:
**06.09.2000 Bulletin 2000/36**

(73) Proprietor: **UOP LLC**
**Des Plaines, IL 60017-5017 (US)**

(72) Inventor: **Gajda, Gregory J.**
**Mount Prospect, Illinois 60056 (US)**

(74) Representative: **Brock, Peter William et al**
**Urquhart-Dykes & Lord,**
**30 Welbeck Street**
**London W1G 8ER (GB)**

(56) References cited:
**DE-C- 550 494** **US-A- 5 723 710**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 033 358 B1

## Description

FIELD

This invention relates to a process for producing monoalkyl aromatic compounds by alkylation. Specifically, this invention relates to highly selective alkylation of benzene by ethylene to produce ethylbenzene using zeolite beta.

BACKGROUND

Alkylation of aromatic compounds with a $C_2$ to $C_4$ olefin is a common reaction for producing monoalkyl aromatic compounds. An example of this reaction that is practiced industrially is the alkylation of benzene with ethylene to produce ethylbenzene. As is usual, several by-products accompany ethylbenzene production. A simplified summary of the alkylation reaction and its common product and by-products is given below:

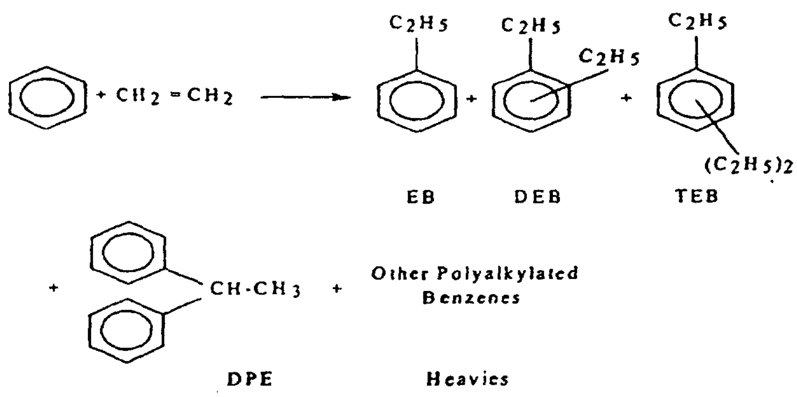


Although the formation of the diethylbenzene ("DEB") and triethylbenzene ("TEB") by-products might, at first glance, be viewed as a reduction in the efficient utilization of ethylene, in fact each can be readily transalkylated by benzene to produce ethylbenzene ("EB"), as shown below:

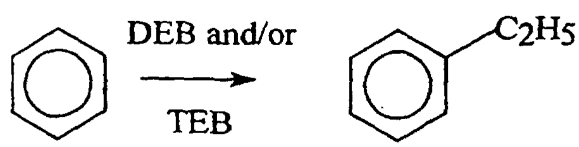


Combining alkylation and transalkylation can thus maximize ethylbenzene production. Such a combination can be carried out in a process having two reaction zones, one for alkylation and the other for transalkylation, or in a process having a single reaction zone in which alkylation and transalkylation both occur. In many cases, a single reaction zone is preferred over two reaction zones because of the savings in capital investment.

In contrast to diethylbenzene and triethylbenzene, 1,1-diphenylethane (1,1-DPE) can not be converted to ethylbenzene by alkylation, and thus 1,1-DPE represents a reduction in ethylene utilization efficiency and a loss of ethylene. In fact, the by-production of 1,1-DPE and of the heavier polyethylated benzenes other than diethylberizene and triethylbenzene which are collectively referred to herein as heavies represents virtually all of the reduction in the ethylene utilization.

Although the ideal ethylbenzene process would form no 1,1-DPE by-product, the current minimum requirement is that the amount of 1,1-DPE formed be not more than 1.0 wt-% of the alkylation reactor effluent relative to ethylbenzene. The formation of 1,1-DPE by-product is assuming added importance and significance in view of the expectation in some areas of near-term minimum standards for the content of 1,1-DPE of not more than 0.5 wt-%.

It is useful to point out two key operating variables of ethylbenzene alkylation zones. The first key variable is the molar ratio of phenyl groups per ethyl group, which is often referred to herein as the phenyl/ethyl ratio. The numerator of this ratio is the number of moles of phenyl groups passing through the alkylation zone during a specified period of time. The number of moles of phenyl groups is the sum of all phenyl groups, regardless of the compound in which the

phenyl group happens to be. For example, one mole of benzene, one mole of ethylbenzene, and one mole of diethylbenzene each contribute one mole of phenyl group to the sum of phenyl groups. The denominator of this ratio is the number of moles of ethyl groups passing through the alkylation zone during the same specified period of time. The number of moles of ethyl groups is the sum of all ethyl and ethenyl groups, regardless of the compound in which the ethyl or ethenyl group happens to be. For example, one mole of ethylene and one mole of ethylbenzene each contribute one mole of ethyl group to the sum of ethyl groups, whereas one mole of diethylbenzene contributes two moles of ethyl groups.

The second key variable is the effective concentration of ethylene entering the alkylation zone. A practical, mathematical approximation is that the concentration of ethylene depends on the reciprocal of the molar ratio of phenyl groups per ethyl group according to the formula:

$$[\text{ethylene}] \approx [\text{phenyl/ethyl ratio}]^{-1}.$$

Thus, increasing the phenyl/ethyl ratio decreases the concentration of ethylene.

It is known that a low concentration of ethylene or a high molar ratio of phenyl groups per ethyl group minimizes formation of 1,1-DPE by-product. The amount of 1,1-DPE formed depends on the square of the reciprocal of the phenyl/ethyl ratio according to the formula:

$$[\text{1,1-DPE}] \approx [\text{phenyl/ethyl ratio}]^{-2}.$$

Thus, increasing the phenyl/ethyl ratio decreases the amount of 1,1-DPE formed. Although the decrease in 1,1-DPE formation that is conferred by a small increase in phenyl/ethyl ratio may be small, it also is very significant, resulting in a high phenyl/ethyl ratio being the condition of choice for minimizing 1,1-DPE formation. However, a high phenyl/ethyl ratio increases capital and operating costs that are usually associated with the recovery of excess benzene. These costs give impetus to a search for an ethylbenzene process that minimizes 1,1-DPE by-product formation at a low phenyl/ethyl ratio.

In the prior art, this search for a commercially-viable ethylbenzene process that not only produces a small amount of 1,1-DPE but also operates at a low phenyl/ethyl ratio has not been fruitful. All of the prior art processes follow the same, well-known approach of dividing the alkylation zone into more and more catalyst beds and injecting smaller and smaller portions of the total ethylene into each bed. Where the allowed concentration of 1,1-DPE is relatively high, this approach undoubtedly confers some benefits. For example, if benzene is alkylated with ethylene in a single-bed alkylation zone that operates at a phenyl/ethyl molar ratio of 5, then the highest concentration of ethylene, which occurs at the point of ethylene injection is 16.7 mol-%. Downstream of the ethylene injection point, the ethylene concentration decreases to very low concentrations as the ethylene is consumed and ethylbenzene is formed, while the phenyl/ethyl ratio remains essentially the same. However, if the single bed is divided into four beds in series and if one-fourth of the required ethylene is injected into each bed, then the phenyl/ethyl ratio is 20 in the first bed, 10 in the second bed, 6.7 in the third bed, and 5 in the fourth bed. Accordingly, the highest concentration of ethylene is 4.8 mol-% in the first bed, 4.5 mol-% in the second bed, and 4.3 mol-% in the third bed, and 4.2 mol-% in the fourth bed. Thus, dividing the bed and splitting the ethylene injection increases the phenyl/ethyl ratio and decreases the highest ethylene concentration.

But, in order to operate at the low phenyl/ethyl ratios and to also attain the low concentrations of 1,1-DPE that are expected to become the minimum standard in the near future, this prior art approach is not viable. For example, if benzene is alkylated with ethylene in a four-bed alkylation zone that operates at an overall phenyl/ethyl molar ratio of 2 rather than 5 as in the previous example, then the phenyl/ethyl ratio ranges from 8 in the first bed to 2 in the fourth bed, and the highest ethylene concentration ranges from 11.1 mol-% in the first bed to 8.3 mol-% in the fourth bed. Compared to the previous example, the ethylene concentration in each bed approximately doubled, which would result in an unacceptable amount of 1,1-DPE formation. In order to reduce the ethylene concentrations to those in the previous example, the number of beds would have to be increased from 4 to 10, simply as a consequence of the fact that the overall phenyl/ethyl ratio had decreased from 5 to 2.

Thus, in response to industry's demand for lower phenyl/ethyl ratios and the market's demand for low 1,1-DPE by-product concentrations in the product stream, the art process inexorably divides the alkylation reaction zone into a large number of very small catalyst beds. Because of a variety of technical, economic, and practical considerations, this inefficient solution by the prior art processes is unacceptable in the hydrocarbon processing industry.

US-A-5 723 710 describes a process for the preparation of a surface-modified zeolite beta for the alkylation and transalkylation of aromatic compounds. The zeolite beta is prepared by treating a templated zeolite beta with acid at a pH between zero and 125°C for a period that leads to a surface aluminum binding energy of at least 74.8 electron

volts.

DE 550 494 C indicates that polycyclic aromatic hydrocarbons can be alkylated in the presence of non-reactive solvents, such as decahydronaphthalene, using such catalysts as metal chlorides and fullers earth.

A method has been discovered to reduce significantly the formation of 1,1-DPE by-product in the alkylation of benzene with ethylene using zeolite beta at a low molar ratio of phenyl groups per ethyl group (phenyl/ethyl ratio). It has also been discovered that this method can significantly reduce the formation of 1,1-DPE in the production of ethylbenzene by alkylation and transalkylation using zeolite beta at a low molar ratio of phenyl groups per ethyl group (phenyl/ethyl ratio). This invention can use one or more components or portions of the alkylation zone effluent stream to dilute the ethylene concentration in the alkylation zone and consequently to decrease the 1,1-DPE by-product formation. This result using zeolite beta was surprising and was not predictable from the prior art, which teaches that 1,1-DPE formation can be reduced only by increasing the phenyl/ethyl ratio or by increasing the number of catalyst beds. Moreover, prior art processes using Y zeolite produce more 1,1-DPE and deactivate more rapidly as a result of using the same diluent components and streams that confer benefits on this invention which uses zeolite beta. A process of alkylating benzene by ethylene at a low ethylene concentration shows a significant selectivity advantage over one operating at a high ethylene concentration. By using this invention, ethylbenzene processes can now minimize 1,1-DPE formation even when operating at low molar ratios of phenyl groups per ethyl group. With the problem of 1,1-DPE formation now solved by this invention, ethylbenzene processes can now operate more profitably at a low molar ratio of phenyl groups per ethyl group.

The underlying chemistry believed responsible for the observed results is that in the alkylation of an aromatic by an olefin, when the concentration of an olefin decreases, there is a selective decrease in the reaction between the olefin and the alkyl aromatic. The products of this reaction are an alkenyl aromatic and a paraffin that correspond to the olefin. The alkenyl aromatic can in turn serve as an active alkylating agent and react with the aromatic to form the undesired by-product diarylalkane. The consequence is that one can expect a decrease in the olefin concentration to confer benefits generally upon the alkylation of aromatics with olefins. Applying this hypothesis to the alkylation of benzene with ethylene, the apparently anomalous formation of 1,1-DPE probably results from the following reactions:

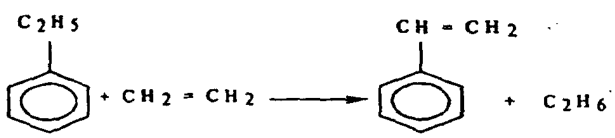

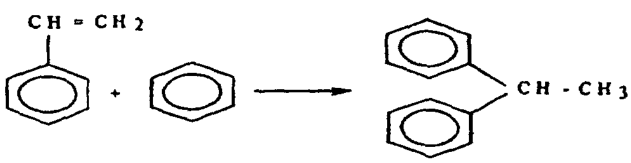

Where a catalyst is used, it is believed that the ethylbenzene and the styrene are chemisorbed on the catalyst, and that hydrogen transfer occurs from the ethylbenzene to ethylene. In any event, a decrease in the concentration of ethylene affords a decrease in the formation of styrene and in turn that of 1,1-DPE.

This invention minimizes 1,1-DPE formation by using a diluent in the combined feed to the alkylation reaction zone to prevent the ethylene concentration from ever attaining the high ethylene concentrations that are present in prior art processes. It is generally known that in prior art processes the concentration of ethylene in the reaction zone decreases from a relatively high concentration at the inlet point where ethylene is introduced to a relatively low concentration at the outlet where nearly all of the ethylene has been consumed. So, even in the prior art processes, low concentrations of ethylene can occur, especially near the outlet of the reaction zone. However, it has been discovered that even the localized high ethylene concentrations that occur in prior art processes at the point of ethylene injection produce unacceptably high concentrations of 1,1-DPE. Thus, it is now recognized that a diluent can preclude localized high ethylene concentrations and minimize 1,1-DPE formation. Moreover it has been recognized that some diluents are preferred over other diluents and that selective choice of this diluent can decease not only the molar ratio of phenyl groups per ethyl group but also the formation of other undesirable by-products besides 1,1-DPE.

This chemistry explains the formation of other diarylalkanes that correspond to other olefins alkylating other aromatics. For example, in the alkylation of benzene with propylene to produce cumene, the corresponding diarylalkane

would probably be 2,2-diphenylpropane (2,2-DPP). Although formation of 1,1-DPP is also possible, 2,2-DPP formation is believed to be more probable because of isomerization of the propyl group.

It is a broad object of this invention to improve the selectivity of and to decrease the costs of processes for the alkylation of aromatics with olefins. It is another broad object of this invention to improve the selectivity of and to decrease the costs of processes for the alkylation of aromatics with olefins and the transalkylation of aromatics with polyalkyl aromatics. It is a specific object of this invention to minimize the formation of 1,1-diphenylethane (1,1-DPE) in alkylation processes that produce ethylbenzene. It is another specific object of this invention to decrease costs associated with operating alkylation processes by decreasing the molar ratio of phenyl groups per alkyl groups at alkylation conditions.

In a broad embodiment, this invention is a process for producing a monoalkylaromatic. A feed aromatic, an olefin, and a diluent comprising at least one phenyl group and at least one alkyl group corresponding to the olefin are passed to an alkylation zone. The feed aromatic and the olefin are reacted in the alkylation zone in the presence of a zeolite beta catalyst to alkylate the aromatic with the olefin to form a monoalkylaromatic. The reaction conditions that inhibit the formation of the diarylalkane by-product are a molar ratio of phenyl groups per atkyl group corresponding to the olefin of from 0.75:1 to 25:1 and a concentration of the olefin, based on the weight of the feed aromatic, the defin, and the diluent passed to the alkylation zone, of less than

$$\frac{MW_O}{6.17 \times MW_A + MW_O} \text{ ,wt-\%}$$

where $MW_0$ is the molecular weight of the olefin and $MW_A$ is the molecular weight of the aromatic.

In a more specific embodiment, this invention is a process for producing ethylbenzene. Benzene, ethylene, and a diluent comprising at least one phenyl group and at least one ethyl group are passed to an alkylation zone. Benzene and ethylene react in the alkylation zone in the presence of a zeolite beta catalyst at reaction conditions sufficient to alkylate benzene with ethylene to form ethylbenzene. The reaction conditions that inhibit the formation of the undesired 1,1-diphenylethane by-product are a molar ratio of phenyl groups per ethyl group of from 1:1 to 6:1 and a concentration of ethylene of less than 5.5 wt-% based on the weight of benzene, ethylene and the diluent passed to the alkylation zone. A product comprising ethylbenzene and the diluent is withdrawn from the alkylation zone. The product contains less than 1.0 wt-% 1,1-diphenylethane relative to ethylbenzene. The diluent is separated from the product and recycled to the alkylation zone.

In another more specific embodiment, this invention is a process for the production of ethylbenzene. Ethylene, an input stream comprising benzene, and a recycle stream containing a diluent are combined to form a combined stream, and the combined stream passes to a reaction zone. The reaction zone contains a catalyst comprising zeolite beta and operates at reaction conditions sufficient to alkylate benzene with ethylene. The reaction conditions inhibiting the formation of the undesired 1,1-diphenylethane are a molar ratio of phenyl groups per ethyl group of from 1:1 to 6:1 and a concentration of ethylene of less than 5.5 wt-% based on the weight of the combined stream. An effluent stream comprising benzene, ethylbenzene, a diethylbenzene, and a heavy polyalkylaromatic is recovered from the reaction zone. The effluent stream contains less than 1.0 wt-% 1,1-diphenylethane relative to ethylbenzene. At least a portion of the effluent stream is passed to a separation zone where the effluent stream is separated. Three streams are withdrawn from the separation zone: a low-boiling fraction comprising benzene, a product stream comprising ethylbenzene, and a high-boiling fraction comprising diethylbenzene and the heavy polyalkylaromatic. The product stream is recovered from the process. A portion of the input stream is provided from at least a portion of the low-boiling fraction. The recycle stream is formed from a portion of the effluent stream or at least a portion of the high-boiling fraction.

In another embodiment, this invention is a process for producing a monoalkyl aromatic. A feed aromatic, an olefin, and a polyalkyl aromatic comprising at least one phenyl group and at least two more alkyl groups corresponding to the olefin than the feed aromatic are passed to a reaction zone. In the reaction zone, the feed aromatic is alkylated with the olefin and the aromatic is transalkylated with the polyalkyl aromatic in the presence of zeolite beta to form a monoalkyl aromatic. The by-product inhibiting reaction conditions include a molar ratio of phenyl groups per alkyl group of from 0.75:1 to 25:1 and a concentration, based on the weight of the feed aromatic the defin, and the polyalkyl aromatic passed to the reaction zone, of the olefin of less than

$$\frac{MW_O}{6.17 \times MW_A + MW_O} \text{ ,wt-\%}$$

wherein $MW_O$ is the molecular weight of the olefin and $MW_A$ is the molecular weight of the aromatic. These reaction conditions inhibit the formation of diarylalkane corresponding to the olefin.

DETAILED DESCRIPTION

This invention can be expected to be applicable generally to the alkylation of an alkylation substrate with an alkylation agent in the presence of a diluent. This invention is more specifically applicable to the alkylation of an aromatic with an olefin. Although benzene is the principal aromatic of interest, aromatics such as alkylsubstituted benzenes, condensed ring systems generally, and alkylated derivatives thereof may be used. Examples of such aromatics are toluene, ethylbenzene, propylbenzene, and so forth; xylene, mesitylene, methylethylbenzene, and so on; naphthalene, anthracene, phenanthrene, methylnaphthalene, dimethylnaphthalene, and tetralin. More than one aromatic can be used.

Olefins containing from 2 to 6 carbon atoms are the principal alkylating agents contemplated for this invention. Examples of such olefins include ethylene, propylene, butene-1, cis-butene-2, trans-butene-2, and iso-butene. However, olefins having from 2 to 20 carbon atoms may be used effectively in this invention. More than one olefin may be used.

The most widely practiced hydrocarbon conversion process to which the present invention is applicable is the catalytic alkylation of benzene with ethylene to produce ethylbenzene. Therefore for purposes of simplification, the discussion herein of the present invention will in large part refer to its application to a catalytic ethylbenzene reaction system.

The diluent may be any compound that is capable of mixing with the alkylating agent (e.g., ethylene) and decreasing the concentration of the alkylating agent at and downstream of the alkylating agent injection point. It is not necessary that the diluent be inert. In practice, however, the diluent should have a number of possible characteristics that are consistent with the process objective of producing high yields of high-purity product. First, the diluent should decrease the molar ratio of phenyl groups to ethyl groups in the reaction zone. Benzene, although suitable, is not preferred because of the high cost of recovering and recycling benzene. Second, the diluent should not adversely affect ethylbenzene yield. Toluene and cumene are not preferred because ethylene can alkylate toluene or cumene and produce by-products that cannot be converted readily to ethylbenzene by alkylation or transalkylation. Ethylbenzene is also not preferred, because ethylbenzene can shift the equilibrium of the alkylation reaction away from the formation of ethylbenzene and because ethylbenzene can react with ethylene to produce styrene and ultimately 1,1-DPE. Third, the diluent should not adversely affect ethylbenzene purity. Xylenes are consequently not preferred because they are relatively difficult to separate from ethylbenzene by distillation. Another reason that xylenes are not preferred is that they can adversely affect ethylbenzene yield by alkylating with ethylene. A fourth characteristic of the diluent, aside from its effect on minimizing 1,1-DPE formation, is that the diluent should increase ethylbenzene yield. Helium, neon, argon, or inert materials are thus not preferred because they cannot react to form ethylbenzene. On the other hand, a reactive diluent or transalkylation agent such as a polyethylbenzene like diethylbenzene, triethylbenzene, and so forth up to even hexaethylbenzene, is preferred because each can transalkylate to ethylbenzene, regardless of whether each is alkylated by ethylene. Because of the possibility of alkylation of the polyethylbenzene by ethylene, however, the lighter polyethylbenzenes are more preferred over the heavier polyethylbenzenes, with diethylbenzene being most preferred.

Where the aromatic is benzene and the olefin is ethylene, the diluent can generally be an alkylbenzene having at least one $C_2$ or one $C_4$ alkyl group. Such alkylbenzenes include ethylbenzene, a diethylbenzene, a triethylbenzene, a butylbenzene, a dibutylbenzene, a tributylbenzene, an ethylbutylbenzene, a diethylbutylbenzene, or diphenylethane. Depending on the particular aromatic and olefin, however, the diluent can be an alkylated derivative of benzene, naphthalene, anthracene, and tetralin.

The reaction conditions inhibiting the formation of the undesired diphenylalkane by-product include a molar ratio of phenyl groups per alkyl group of from 25:1 to 0.75. In this invention, successful operation at low molar ratio of phenyl groups per ethyl group, e.g., below 6:1, is achieved by introducing the diluent to the reaction zone so that the concentration of ethylene remains less than 5.5 wt-%. By contrast, in prior art processes for the alkylation of benzene with ethylene, if the molar ratio of phenyl groups per ethyl group is 6.17, then ethylene constitutes 5.5 wt-% of the total weight of hydrocarbons, namely benzene and ethylene, that is passed to the alkylation reaction zone. In the general case for alkylating agents other than ethylene and alkylation substrates other than benzene, successful operation in accord with this invention at a low molar ratio of phenyl groups per alkyl group is achieved by introducing the diluent to the reaction zone so that the concentration, based on the weight of alkylating agent, alkylation substrate and diluent passed to the alkylation zone, in weight percent of alkylating agent remains less than that computed by the following formula:

$$\frac{MW_{AA}}{6.17 \; x \; MW_{AS} + MW_{AA}} \; ,wt\text{-}\%,$$

where $MW_{AA}$ is the molecular weight of the alkylation agent (e.g., olefin) and $MW_{AS}$ is the molecutar weight of the alkylation substrate (e.g., benzene).

In general, for a given molar ratio of alkylation substrate per alkylation agent, the greater the molar ratio of phenyl groups to alkyl groups in the feed stream, the less is the rise in temperature in the reaction zone that occurs as a result of the alkylation reactions. The alkylation reactions have a heat of reaction of 233 to 349 kJlkmole (100-150 BTU/lb-mole) and are considered to be moderately exothermic. Although some ethylbenzene reactors have indirect heat exchange means to remove the heat as it is produced, most ethylbenzene reactors are adiabatic, and so the outlet temperature of the effluent stream is higher than the inlet temperature of the reactants. An increase in the molar ratio of phenyl groups to alkyl groups in the feed stream increases the quantity of phenyl groups available to act as a heat sink in the reaction zone and thus decreases the temperature rise in the reaction zone. Thus, in practicing this invention, the inlet temperature in the reaction zone is typically from 200 to 260°C (392 to 500°F) and preferably from 230 to 250 °C (446 to 482°F). Although the temperature rise that occurs in the reaction zone could be from 10 to 190°C (18 to 342°F) depending on the total mass flows in the reactor, the temperature rise is generally from 5 to 50°C (9 to 90 °F), and preferably from 5 to 20°C (9 to 36°F). In general, for all reactants taught herein the appropriate reaction temperature is generally from 100°C (212°F) to the critical temperature of the alkylation substrate, which may be 475° C (887°F) or even higher.

The temperature rise in the reaction zone may be controlled by adjusting the molar ratio of phenyl groups to ethyl groups in the feed stream. Minimizing the temperature rise helps prevent high reactor outlet temperatures, which cause undesirable side reactions such as cracking of hydrocarbons to occur. High reaction temperatures can also cause vaporization of benzene and ethylbenzene in the reaction zone. In one embodiment of this invention, the temperature rise in the reaction zone can be controlled by withdrawing an effluent stream from the reaction zone, cooling a portion of the effluent stream, and recycling the cooled portion of the effluent stream to the reaction zone. Although recycling reactor effluent to the reaction zone in this manner may be disadvantageous for some reaction zones, it is not disadvantageous for this invention because recycling reactor effluent to the reaction zone does not significantly alter the product distribution when the catalyst is zeolite beta. A significant alteration in the product distribution is a change in the concentration of any of the products in the reactor effluent stream of more than 0.5 wt-%. A significant alteration in the product distribution does not occur because at the reaction conditions zeolite beta is such an active promoter of the alkylation reaction between benzene and ethylene that the extent of reaction proceeds at least 80% and generally more than 90% of the way to equilibrium. Thus, recycling reactor effluent to the reaction zone does not interfere in a significant way with the extent of the alkylation reaction, and recycling reactor effluent may be employed for the purpose of controlling reaction zone temperatures.

The alkylation is performed in the liquid phase. Consequently, reaction pressure needs to be sufficiently high to ensure at least a partial liquid phase. Where ethylene is the olefin, the pressure range for the alkylation reaction is usually from 1379 to 6985 kPa(g) (200 to 1000 psig), more commonly from 2069 to 4137 kPa(g), and even more commonly from 3103 to 4137 kPa(g). Preferably, the reaction conditions are sufficient to maintain benzene in a liquid phase and are supercritical conditions for ethylene. For olefins other than ethylene, this invention may be generally at a pressure of from 345 to 6985 kPa(g) (50 to 1000 psig).

The weight hourly space velocity of ethylene may range from 0.01 to 2.0 hr-1. The weight hourly space velocity of aromatics, including benzene and an aromatic diluent having at least one $C_2$+ group, is generally from 0.3 to 480 $hr^{-1}$. In a preferred embodiment, in which the diluent is a diethylbenzene or a triethylbenzene, the molar ratio of benzene per ethylene is from 2:1 to 6:1, the weight hourly space velocity of ethylene is from 0.1 to 1.0 $hr^{-1}$, and the weight hourly space velocity of aromatics, including benzene and the diluent is from 0.5 to 19 $hr^{-1}$.

The principal reaction that occurs in the reaction zone is the alkylation of the benzene by ethylene to produce the ethylbenzene. In addition, other reactions can occur in the reaction zone. For example, the diluent can be alkylated with ethylene or with ethylbenzene, or the diluent can transalkylate with benzene or with ethylbenzene. Although the extent to which these other reactions form by-products is diminished by the practice of this invention, the reactor effluent stream nevertheless usually contains the by-products of these other reactions. Accordingly, a portion of the reactor effluent stream can be used without any downstream separation as a stream for supplying diluent to the alkylation reaction zone. Alternatively, the reactor effluent stream can be passed to a separation zone from which can be recovered a fraction containing one or more components that are suitable diluents, and this fraction can in turn be passed to the alkylation reaction zone.

The reactor effluent stream contains ethylbenzene and may also contain unreacted diluent, a by-product of an alkylation side reaction involving the diluent, or a by-product of a transalkylation side reaction involving the diluent. The reactor effluent stream may also contain unreacted benzene as well as a by-product of an alkylation side reaction involving benzene or a by-product of a transalkylation side reaction involving benzene. In addition, the reactor effluent stream may contain unreacted ethylene, but the concentration of unreacted ethylene is likely to be insignificant because benzene is usually present at least in a stoichiometric proportion. Although it is not common for the feed stream to contain $C_1$ to $C_3$ paraffins in addition to ethylene, if ethane is present in the feed stream then the reactor effluent stream

may also contain unreacted ethane.

The reactor effluent stream passes to a separation zone, which generally comprises a benzene fractionation column in order to recycle unreacted benzene to the alkylation zone, and an ethylbenzene fractionation column in order to recover ethylbenzene as product from the heavier polyalkylbenzenes. A polyalkylbenzene fractionation column may also be used in order to separate diethylbenzenes and triethylbenzenes from the other heavier polyalkylbenzenes, particularly where the alkylbenzene that is present in the feed stream is a diethylbenzene or a triethylbenzene. The separation zone generally does not comprise a deethanizer unless the concentrations of unreacted ethylene, ethane, or light $C_3$- paraffins in the reactor effluent are high enough to justify their being separated from the reactor effluent stream.

Zeolite beta is disclosed in US-A-3308069, US-A-4891458 and US-A-5081323; and a steamed and ammonium exchanged zeolite beta is disclosed in US-A-5522984.

US-A-3308069 discloses a technique for the manufacture of zeolite beta by heating amorphous silica solids or sols with a soluble aluminate and tetraethylammonium hydroxide in an aqueous medium at a temperature of 75 to 200°C. The aluminate may be sodium or tetraethylammonium aluminate, and amorphous silica-alumina may be used as the source of silica and alumina. The zeolite beta thus formed may be activated by calcining at about 205-930°C [400-1700°F].

US-A-4891458 and US-A-5081323 disclose a similar process for making zeolite beta and the use of the zeolite beta thus formed in the alkylation or transalkylation of aromatic compounds with olefins or polyalkylaromatic hydrocarbons.

US-A-5522084 discloses a modified zeolite beta with an enhanced activity for the transalkylation of diisopropyl benzene. The modification process involves steam treating zeolite beta at 500-800°C and subsequently treating the product with a source of ammonium ions.

A preferred zeolite beta for use in this invention is a surface-modified zeolite beta which results from acid washing of a templated native zeolite beta. That is, the formation of the surface-modified zeolite beta starts with a templated beta where the template is, for example, a tetraalkylammonium salt, such as tetraethylammonium salt. It is critical to acid wash a templated zeolite beta in order to protect the internal sites of the zeolite and to prevent dealumination. The templated zeolite beta is treated with a strong acid at a pH between 0 up to 2, although a pH under 1 is preferred. Acids which may be used include nitric acid, sulfuric acid, phosphoric acid, and so forth. For example, a weak, 0.01 molar nitric acid may be used in conjunction with ammonium nitrate to perform the acid wash, although substantially higher concentrations, up to 20 weight percent nitric acid, are preferred. Nitric acid is a preferred acid since it is a non-complexing acid and therefore does not encourage dealumination. Treatment of the templated zeolite beta with strong acid may be effected over the temperature range between 20°C up to 125°C. It is important that acid washing be done under conditions not so severe as to effect dealumination. The time over which acid washing is conducted in preparing the preferred zeolite is quite temperature dependent. It is critical in the formation of the surface-modified zeolite beta that there be no significant bulk dealumination of the zeolite. Thus, as a general statement it can be said that acid washing should be done for a time insufficient to effect dealumination. For example, using 0.01 molar nitric acid and 40% ammonium nitrate at 70°C, contact times of 2-3 hours are found adequate to modify the environment of surface aluminum without causing significant bulk dealumination. Using 15% nitric acid with ammonium nitrate to treat a 25 weight percent slurry at 85°C, a 90-minute treatment is effective. The template is removed by calcination at temperatures in the range of 550-700°C. Calcination conditions are well known in the art and need not be elaborated upon here. It also needs to be mentioned that powdered zeolite itself is not usually used as the alkylation catalyst. Therefore, in the more usual case after the templated zeolite beta is acid washed it is mixed with a conventional binder, extruded, and the extrudate is ultimately calcined. But the critical portion of the preparation of the preferred zeolite is the acid wash of the templated beta according to the foregoing description. Acid washing a calcined (i.e., non-templated) zeolite beta does not afford the surface-modified material of the preferred zeolite.

It has been found that after treatment as described above the surface aluminum atoms are chemically modified. It has been hypothesized that the modification is in the form of replacement of strong acid sites at the catalyst surface by weaker acid sites. What has been definitely observed is that the surface aluminums of the preferred modified zeolite beta have 2p binding energies as measured by x-ray photoelectron spectroscopy of at least 74.8 electron volts. See US-A-5723710 for additional details.

## EXAMPLES

Catalyst A is fresh alkylation catalyst comprising zeolite beta made in accordance with the teachings of US-A-3308069.

A sample of Catalyst A was used to produce ethylbenzene by alkylating benzene with ethylene at alkylation conditions at which heavy alkylaromatics were occluded on the surface and within the internal pore space of the sample of Catalyst A. After having been used for alkylation, the sample of Catalyst A had a content of occluded heavy alkylaromatics of 5 % by weight of the catalyst weight. While being contacted with air, the sample of Catalyst A having occluded heavy alkylaromatics was heated from ambient temperature to 650°C (1202°F) over a period of three hours, was maintained at 650°C for three hours, and then was cooled to room temperature. The sample of Catalyst A after cooling is referred to in these Examples as Catalyst B.

Catalyst C is a fresh alkylation catalyst comprising zeolite beta made in accordance with US-A-3308069.

Catalyst D is a fresh alkylation catalyst comprising 80 wt-% 'ultrastabilized' zeolite Y and 20 wt-% alumina binder.

In the Examples 1-9 that follow, the net reactor effluent stream is the total reactor effluent stream less the portion, if any, of the total reactor effluent stream that is recycled to the reactor. Efficiency is defined with respect to ethylene and is computed by subtracting the weight of ethylene in the net reactor effluent stream from the weight of ethylene in the make-up ethylene to the reactor, divided by the weight of ethylene in the net reactor effluent stream, times 100. Selectivity of 1,1-DPE is defined as the concentration in weight percent of 1,1-DPE in the net reactor effluent stream, computed on the basis of the net reactor effluent stream being free of benzene and light compounds. In general, the yield of a compound is defined as the product of conversion and selectivity of that compound, divided by 100. However, the ethylbenzene yield has a special definition in that it is defined as the sum of the individual yields of ethylbenzene, diethylbenzene, and triethylbenzene. This computation of the ethylbenzene yield accounts for the total yield of ethylbenzene that would be produced if all the diethylbenzene and triethylbenzene in the product stream was transalkylated to ethylbenzene in a transalkylation zone and subsequently recovered.

In addition, in the Examples 1-9 the benzene liquid hourly space velocity (LHSV) is computed using only the make-up benzene and does not include the benzene in the portion, if any, of the total reactor effluent stream that is recycled to the catalyst bed. Also, because the molar ratio of phenyl groups per ethyl group (or per propyl group) is essentially the same in the total reactor feed stream and the total reactor effluent stream, the molar ratio of phenyl groups per ethyl group (or per propyl group) is not significantly affected by recycling any portion of the total reactor effluent stream.

In Examples 1-7, the catalyst is contacted with a combined feed stream containing fresh benzene, fresh ethylene, a recycled aliquot portion of the reactor effluent stream (in Examples 1, 3, 5, 6, and 7 only), and fresh diethylbenzene (in Example 6 only). Where a portion of the reactor effluent stream is recycled to the reactor, the ratio of the weight of the recycled portion of the reactor effluent stream per the weight of fresh benzene and fresh ethylene was 2.0. The ethylene concentration in the combined feed is more than 5.5 wt. % in Examples 2, 4 and 5, which are the control examples.

Table 1

Effect of Ethylene Concentration on 1,1-DPE Formation at Various Molar Ratios of Phenyl Groups per Ethyl Group using Zeolite Beta Catalysts

| Example | 1 | 2 | 3 | 4 | 5 | 6 | 7 |
|---|---|---|---|---|---|---|---|
| Catalyst | B | B | B | B | B | A | A |
| Phenyl/Ethyl, mol/mol | 5.21 | 5.25 | 4.09 | 4.27 | 1.81 | 4.54 | 4.55 |
| Maximum temp., °C | 241.9 | 252.5 | 201.7 | 252.1 | 231.8 | 242 | 242 |
| Temp. rise, °C | 15.9 | 33.3 | 20.9 | 34.7 | 24.1 | 23.4 | 25.5 |
| Pressure, mPa | 3.89 | 3.89 | 3.89 | 3.89 | 3.89 | 3.89 | 3.89 |
| Benzene LHSV, $hr^{-1}$ | 3.9 | 4.1 | 2.6 | 3.3 | 1.3 | 3.9 | 2.9 |
| Recycle effluent/fresh feed, wt/wt | 2.0 | 0 | 2.0 | 0 | 2.0 | 2.0 | 2.0 |
| Ethylene concentration in combined feed wt-% | 2.15 | 6.4 | 2.79 | 7.75 | 5.52 | 1.88 | 2.44 |
| EB yield, wt-% | 99.82 | 99.79 | 99.83 | 99.73 | 98.24 | 99.49 | 99.38 |
| 1,1-DPE selectivity, wppm | 886 | 928 | 895 | 1230 | 11180 | 2900 | 3100 |
| 1,1-DPE/EB in reactor effluent (wt-%/wt-%) $x10^2$ | .101 | .105 | .105 | .143 | 1.74 | .357 | .348 |

A comparison of Examples 1 and 2 shows the effect of recycling a portion of the reactor effluent stream to the reactor in order to decrease the concentration of ethylene in the feed stream at nearly the same molar ratio of phenyl groups per ethyl group (5.21 and 5.25). Recycling a portion of the effluent stream in Example 1 increases the

ethylbenzene yield, decreases the 1,1-DPE selectivity, and decreases the ratio of 1,1-DPE per ethylbenzene in the effluent stream.

A comparison of Examples 3 and 4 shows the effect of recycling a portion of the reactor effluent stream to the reactor in order to decrease the concentration of ethylene in the feed stream at molar ratios of phenyl groups per ethyl group (4.09 and 4.27) that are lower than in Examples 1 and 2. Recycling a portion of the reactor effluent stream in Example 3 increases the ethylbenzene yield, decreases the 1,1-DPE selectivity, and decreases the ratio of 1,1-DPE per ethylbenzene in the effluent stream, even though the molar ratio of phenyl groups per ethyl group is lower in Example 3 than in Example 4.

Example 5 shows the effect of recycling a portion of the reactor effluent stream to the reactor at a lower molar ratio of phenyl groups per ethyl group than in Examples 1 through 4, but without decreasing the concentration of ethylene in the feed stream. With the concentration of ethylene in the feed stream being relatively high at 5.52 wt-%, then even though the molar ratio of phenyl groups per ethyl group is 1.81:1, the ethylbenzene yield is lower, the 1,1-DPE selectivity is higher, and the ratio of 1,1-DPE per ethylbenzene in the effluent stream is higher than in Examples 1 through 4.

In Example 6, fresh diethylbenzene was passed to the reactor in order to simulate the effect of recycling diethylbenzene. Fresh diethylbenzene constituted 4.5 wt-% of the weight of the fresh benzene, fresh ethylene, and fresh diethylbenzene that was passed to the reactor. The molar ratio of phenyl groups per ethyl group of 4.54 takes into account the phenyl and ethyl groups of the fresh diethylbenzene. A comparison of Examples 6 and 7 shows the effect of introducing diethylbenzene to the reactor while recycling a portion of the effluent stream to the reactor at nearly the same molar ratios of phenyl groups per ethyl group. Introducing diethylbenzene increases the ethylbenzene yield and decreases the 1,1-DPE selectivity.

In Examples 8 and 9, the catalyst is contacted with a combined feed stream containing fresh benzene, fresh propylene, and a recycled aliquot portion of the reactor effluent stream. The ratio of the weight of the recycled portion of the reactor effluent stream per the weight of fresh benzene and fresh propylene was 1.5 in Example 8 and 1.75 in Example 9. The position of the maximum temperature (due to the exothermic reaction) in the catalyst bed was noted. Deactivation was determined by noting the position of the maximum temperature after a suitable interval of time (e.g., 48 hours) at test conditions. Deactivation is calculated by taking the difference in these two positions (in inches), dividing by the bed length (in inches), and then dividing by the time interval (in days). The results are multiplied by 100% to give a deactivation rate in percent of catalyst bed/day.

Table 2

| Effect of Recycling Reactor Effluent on Catalyst Deactivation Rate at the Same Phenyl/Propyl Ratio using Zeolite Beta Catalyst | | |
|---|---|---|
| Example | 8 | 9 |
| Catalyst | C | C |
| Phenyl/Propyl, mol/mol | 4 | 4 |
| Maximum temp., °C | 180 | 180 |
| Temp. rise °C | 25 | 23 |
| Pressure, mPa | 3.55 | 3.85 |
| Benzene LHSV, $hr^{-1}$ | 4 | 4 |
| Recycle effluent/fresh feed, wt/wt | 1.5 | 1.75 |
| Propylene concentration in combined feed, wt-% | 4.7 | 4.3 |
| Deactivation rate, %/day | 3.45 | 2.86 |

A comparison of Examples 8 and 9 shows that increasing the recycle ratio of the effluent stream to the catalyst bed at the same molar ratio of phenyl groups per propyl group decreases the rate of catalyst deactivation. Although these data showing a decrease in the deactivation rate were obtained while Catalyst C was used to alkylate benzene with propylene, it is believed that a similar decrease in the rate of catalyst deactivation would be observed if Catalyst C is used to alkylate benzene with ethylene.

Examples 10 and 11, are control examples using zeolite Y in which the catalyst is contacted with a combined stream comprising fresh benzene, fresh ethylene, and a recycled aliquot portion of the reactor effluent stream (in Example 10 only). In Example 10, the ratio of the weight of the recycled portion of the reactor effluent stream per the weight of fresh benzene was 3. Deactivation rates were determined by the calculation method described previously for Examples 8 and 9.

Table 3

| Effect of Recycling Reactor Effluent on 1,1-DPE Formation and on Catalyst Deactivation Rate at the Same Phenyl/Ethyl Ratio Using Zeolite Y Catalyst | | |
|---|---|---|
| Example | 10 | 11 |
| Catalyst | D | D |
| Phenyl/Ethyl, mol/mol | 5.0 | 5.0 |
| Maximum temp., °C | 240 | 240 |
| Pressure, psi(g) | 550 | 550 |
| Ethylene LHSV, $hr^{-1}$ | 0.3 | 0.3 |
| Recycle effluent/fresh benzene, wt/wt | 3 | 0 |
| 1,1-DPE/EB in reactor effluent, (wt-%/wt-%)x$10^2$ | 2.76 | 2.45 |
| Deactivation rate, %/day | 2.5 | 1.7 |

A comparison of Examples 10 and 11 shows that increasing the recycle ratio of the effluent stream to the catalyst bed at the same molar ratio of phenyl groups per ethyl group increases the ratio of 1,1-DPE per ethylbenzene in the effluent stream and increases the rate of catalyst deactivation. Thus, in contrast to zeolite beta, zeolite Y's performance worsens as a result of recycling reactor effluent.

## Claims

**1.** A process for producing a monoalkylaromatic comprising:

a) passing a feed aromatic, an olefin, and a diluent comprising at least one phenyl group and at least one more alkyl group corresponding to said olefin than said feed aromatic to an alkylation zone;

b) reacting said feed aromatic and said olefin in said alkylation zone in the presence of zeolite beta to alkylate said feed aromatic with said olefin to form a monoalkylaromatic;

c) inhibiting the formation of diarylalkane by-product corresponding to said olefin by operating said alkylation zone at reaction conditions comprising a molar ratio of phenyl groups per alkyl group corresponding to said olefin of from 0.75:1 to 25:1 and a concentration of said olefin, based on the weight of said feed aromatic, said olefin, and said diluent passed to said alkylation zone in Step (a), of less than

$$\frac{MW_O}{6.17 \times MW_A + MW_O}, \text{wt-\%}$$

wherein $MW_O$ is the molecular weight of said olefin and $MW_A$ is the molecular weight of said feed aromatic; and

d) withdrawing from said alkylation zone a product comprising said monoalkylaromatic.

**2.** The process of Claim 1 further **characterized in that** said reaction conditions comprise a temperature of from 100°C to 475°C and a pressure sufficient to maintain said feed aromatic in at least a partial liquid phase.

**3.** The process of Claims 1 or 2 wherein said diluent is selected from the group consisting of alkylated derivatives of benzene, alkylated derivatives of naphthalene, alkylated derivatives of anthracene, and alkylated derivatives of tetralin.

**4.** The process of Claims 1 or 2 wherein said feed aromatic is selected from the group consisting of benzene, naphthalene, anthracene, tetralin, and alkylated derivatives thereof.

**5.** The process of Claims 1 or 2 wherein said olefin has from 2 to 20 carbon atoms.

**6.** The process of Claims 1 or 2 wherein said olefin is ethylene, said feed aromatic is benzene, said monoalkylaromatic

is ethylbenzene, said diarylalkane by-product is 1,1-diphenylethane, and said diluent is diethylbenzene.

**7.** The process of Claims 1 or 2 wherein said olefin is propylene, said feed aromatic is benzene, said monoalkylaromatic is cumene, said diarylalkane by-product is 2,2-diphenylpropane and said diluent is dipropylbenzene.

**8.** The process of Claims 1 or 2 wherein said zeolite beta comprises a calcined, non-templated surface-modified zeolite beta **characterized by** having surface aluminum 2p binding energies, as measured by X-ray photoelectron spectroscopy, of at least 74.8 electron volts.

**9.** The process of Claims 1 or 2 further **characterized in that** said olefin is ethylene, said feed aromatic is benzene, said monoalkylaromatic is ethylbenzene, said diarylalkane by-product is 1,1-diphenylethane, and said diluent is selected from the group consisting of ethylbenzene, a diethylbenzene, a triethylbenzene, a tetraethylbenzene, an ethylbutylbenzene, or a diethylbutylbenzene.

**Patentansprüche**

**1.** Verfahren zum Herstellen eines Monoalkylaromaten, umfassend:

(a) das Zuführen eines Einspeisearomaten, eines Olefins und eines Verdünnungsmittels, das mindestens eine Phenylgruppe und mindestens eine dem Olefin entsprechende Alkylgruppe mehr als der Einspeisearomat enthält, zu einer Alkylierungszone;

(b) das Reagieren des Einspeisearomaten und des Olefins in der Alkylierungszone in Anwesenheit von Zeolith Beta zum Alkylieren des Einspeisearomats mit dem Olefin unter Bildung eines Monoalkylaromaten;

(c) das Hemmen der Bildung von dem Olefin entsprechendem Diarylalkan-Nebenprodukt durch Betreiben der Alkylierungszone unter Reaktionsbedingungen, die ein Molverhältnis von Phenylgruppen pro dem Olefin entsprechender Alkylgruppe von 0,75:1 bis 25:1 und eine Konzentration des Olefins, auf das Gewicht des Einspeisearomats, des Olefins und des in Schritt (a) der Alkylierungszone zugeführten Verdünnungsmittels bezogen, von weniger als

$$\frac{MW_O}{6{,}17 \times MW_A + MW_O} \text{Gew.-\%}$$

umfassen, wobei $MW_o$ die Molmasse des Olefins und $MW_A$ die Molmasse des Einspeisearomats ist; und

(d) das Herausnehmen eines den Monoalkylaromat umfassenden Produkts aus der Alkylierungszone.

**2.** Verfahren nach Anspruch 1, des Weiteren **dadurch gekennzeichnet, dass** die Reaktionsbedingungen eine Temperatur von 100 °C bis 475 °C und einen Druck umfassen, der ausreicht, um den Einspeisearomaten in einer mindestens teilweise flüssigen Phase zu halten.

**3.** Verfahren nach Ansprüchen 1 oder 2, wobei das Verdünnungsmittel aus der Gruppe ausgewählt wird bestehend aus alkylierten Derivaten von Benzol, alkylierten Derivaten von Naphthalin, alkylierten Derivaten von Anthracen und alkylierten Derivaten von Tetralin.

**4.** Verfahren nach Ansprüchen 1 oder 2, wobei der Einspeisearomat aus der Gruppe ausgewählt wird bestehend aus Benzol, Naphthalin, Anthracen, Tetralin und alkylierten Derivaten derselben.

**5.** Verfahren nach Ansprüchen 1 oder 2, wobei das Olefin 2 bis 20 Kohlenstoffatome aufweist.

**6.** Verfahren nach Ansprüchen 1 oder 2, wobei das Olefin Ethylen ist, der Einspeisearomat Benzol ist, der Monoalkylaromat Ethylbenzol ist, das Diarylalkan-Nebenprodukt 1,1-Diphenylethan ist und das Verdünnungsmittel Diethylbenzol ist.

**7.** Verfahren nach Ansprüchen 1 oder 2, wobei das Olefin Propylen ist, der Einspeisearomat Benzol ist, der Monoalkylaromat Cumol ist, das Diarylalkan-Nebenprodukt 2,2-Diphenylpropan ist und das Verdünnungsmittel Dipropylbenzol ist.

**8.** Verfahren nach Ansprüchen 1 oder 2, wobei der Zeolith Beta calcinierten, nicht mit Templante behandelten, oberflächenmodifizierten Zeolith Beta umfasst, **dadurch gekennzeichnet, dass** er Aluminium 2p-Oberflächenbindungsenergien, wie sie durch Röntgen-Photoelektronenspektroskopie gemessen werden, von mindestens 74,8 Elektronenvolt besitzt.

**9.** Verfahren nach Ansprüchen 1 oder 2, des Weiteren **dadurch gekennzeichnet, dass** das Olefin Ethylen ist, der Einspeisearomat Benzol ist, der Monoalkylaromat Ethylbenzol ist, das Diarylalkan-Nebenprodukt 1,1-Diphenylethan ist und das Verdünnungsmittel aus der Gruppe ausgewählt wird bestehend aus Ethylbenzol, einem Diethylbenzol, einem Triethylbenzol, einem Tetraethylbenzol, einem Ethylbutylbenzol oder einem Diethylbutylbenzol.

## Revendications

**1.** Procédé de production d'un composé monoalkylaromatique comprenant:

a) le passage, à une zone d'alkylation, d'un composé aromatique d'alimentation, d'une oléfine et d'un diluant comprenant au moins un groupement phényle et au moins un groupement alkyle de plus correspondant à ladite oléfine que ledit composé aromatique d'alimentation;

b) la mise en réaction dudit composé aromatique d'alimentation et de ladite oléfine dans ladite zone d'alkylation en présence de zéolite bêta en vue de procéder à l'alkylation dudit composé aromatique d'alimentation avec ladite oléfine, pour former un composé monoalkylaromatique;

c) l'inhibition de la formation de produit secondaire de diarylalcanes correspondant à ladite oléfine en opérant ladite zone d'alkylation dans des conditions de réaction comprenant un rapport molaire de groupements phényle par groupement alkyle correspondant à ladite oléfine allant de 0,75:1 à 25:1 et une concentration de ladite oléfine, sur la base du poids dudit composé aromatique d'alimentation, de ladite oléfine et dudit diluant, passé à ladite zone d'alkylation dans l'étape (a), de moins de

$$\frac{MW_O}{6{,}17 \times MW_A + Mw_O} \text{ \% en poids}$$

dans lequel $MW_O$ est le poids moléculaire de ladite oléfine et $MW_A$ est le poids moléculaire dudit composé aromatique d'alimentation ; et

d) le retrait de ladite zone d'alkylation d'un produit comprenant ledit composé monoalkylaromatique.

**2.** Procédé selon la revendication 1, **caractérisé en outre en ce que** lesdites conditions de réaction comprennent une température allant de 100°C à 475°C et une pression suffisante à maintenir ledit composé aromatique d'alimentation dans au moins une phase liquide partielle.

**3.** Procédé selon les revendications 1 ou 2, dans lequel ledit diluant est sélectionné parmi le groupe constitué de dérivés alkylés du benzène, de dérivés alkylés du naphtalène, de dérivés alkylés de l'anthracène, et de dérivés alkylés de la tétraline.

**4.** Procédé selon les revendications 1 ou 2, dans lequel ledit composé aromatique d'alimentation est sélectionné parmi le groupe constitué du benzène, du naphtalène, de l'anthracène, de la tétraline, et de dérivés alkylés de ces derniers.

**5.** Procédé selon les revendications 1 ou 2, dans lequel ladite oléfine a de 2 à 20 atomes de carbone.

**6.** Procédé selon les revendications 1 ou 2, dans laquelle ladite oléfine est l'éthylène, ledit composé aromatique d'alimentation est le benzène, ledit composé monoalkylaromatique est l'éthylbenzène, ledit produit secondaire de diarylalcanes est le 1,1-diphényléthane, et ledit diluant est le diéthylbenzène.

7. Procédé selon les revendications 1 ou 2, dans laquelle ladite oléfine est le propylène, ledit composé aromatique d'alimentation est le benzène, ledit composé monoalkylaromatique est le cumène, ledit produit secondaire de diarylalcanes est le 2,2-diphénylpropane, et ledit diluant est le dipropylbenzène.

8. Procédé selon les revendications 1 ou 2, dans lequel ladite zéolite bêta comprend une zéolite bêta calciné, modifié en surface, sans matrice, **caractérisée en** ayant des énergies de liaison 2p d'aluminium de surface, telles qu'elles sont mesurées par spectroscopie photo électronique aux rayons X, d'au moins 74,8 électron-volts.

9. Procédé selon les revendications 1 ou 2, **caractérisé en outre en ce que** ladite oléfine est l'éthylène, ledit composé aromatique d'alimentation est le benzène, ledit composé monoalkylaromatique est l'éthylbenzène, ledit produit secondaire de diarylalcanes est le 1,1-diphényléthane, et ledit diluant est sélectionné parmi le groupe constitué de l'éthylbenzène, d'un diéthylbenzène, d'un triéthylbenzène, d'un tétraéthylbenzène, d'un éthylbutylbenzène ou d'un diéthylbutylbenzène.